# EUROPEAN PATENT SPECIFICATION

(11) **EP 3 038 479 B1**
(45) Date of publication and mention of the grant of the patent: **02.01.2019**
(21) Application number: 13895763.4
(22) Date of filing: 18.10.2013
(51) Int. Cl.: A24F 47/00, A61M 11/04, A61M 15/06, F16B 7/20, F16L 37/107, F16L 37/252, H01R 13/625

(54) **ELECTRONIC VAPOUR PROVISION SYSTEM**
ELEKTRONISCHE DAMPFBEREITSTELLUNGSVORRICHTUNG
SYSTÈME DE FOURNITURE DE VAPEUR ÉLECTRONIQUE

(43) Date of publication of application: 06.07.2016
(73) Proprietor: Nicoventures Holdings Limited, London WC2R 3LA (GB)
(72) Inventor: LEADLEY, David, London WC2R 2PG (GB); DICKENS, Colin, London WC2R 2PG (GB); DING, Yingzhuo, Shenzhen Guangdong 518040 (CN)
(74) Representative: D Young & Co LLP
(86) International application number: PCT/CN2013/085455
(87) International publication number: WO 2015/054885

(56) References cited:
- EP-A1- 2 875 739
- EP-A2- 0 105 810
- WO-A1-2010/118644
- WO-A1-2013/147492
- WO-A1-2014/150773
- WO-A2-2010/140841
- WO-A2-2013/040193
- CN-A- 102 326 869
- CN-U- 202 112 305
- US-A- 4 235 498
- US-A1- 2013 081 642

## Description

### Field

The present disclosure relates to electronic vapour provision systems such as electronic nicotine delivery systems, including e-cigarettes.

### Background

Electronic vapour provision systems such as e-cigarettes generally contain a reservoir of liquid which is to be vaporised, typically nicotine. When a user inhales on the device, a heater is activated to vaporise a small amount of liquid, which is therefore inhaled by the user. Many such devices comprise multiple components which can be separated in an axial direction, for example to replace a cartridge containing a reservoir of nicotine that has now been exhausted and/or to recharge a cell or battery which is used to power the heater. The point of separation between two such components represents a potential weakness in the overall structure of an e-cigarette.
WO 2010 140841 A2 discloses a liquid vaporizing and inhaling apparatus.
WO 2013 040193 A2 discloses an electronic cigarette having a first section housing electrical components.
WO 2010 118644 A1 discloses a heating atomization electric-cigarette adopting capacitor for power supply comprising a power part, a body part and a hollow cigarette holder.

### Summary

The invention is defined in the appended claims.

Some embodiments of the invention provide an electronic vapour provision system that includes a first unit comprising a battery or cell and a second unit comprising a heater and a nicotine source for producing nicotine vapour when heated. The first unit and the second unit comprise a bayonet fitting for providing a mechanical and electrical connection between the first and second unit. The electrical connection allows the battery or cell to provide power to the heater to produce nicotine vapour from the nicotine source. The electronic vapour provision system has a longitudinal axis, and the bayonet fitting is configured such that the first and second units are connected by relative movement of the first and second units along said longitudinal axis to a first engagement position, and relative rotation of the first and second units about the longitudinal axis from the first engagement position to a final engagement position that prevents both (i) relative movement of the first and second units along said longitudinal axis, and (ii) relative rotation of the first and second units further away from the first engagement position. The bayonet fitting further includes a biasing means that provides a rotational bias to prevent rotation of the first and second units from the final engagement position to the first engagement position. A transition point is located between the first engagement position and the final engagement position, and when the second unit is located between the first engagement position and the transition position, the biasing means applies a rotational bias in favour of rotation of the second unit in a rotational direction from the final engagement position towards the first engagement position, and when the second unit is located between the final engagement position and the transition position, the biasing means applies a rotational bias in favour of rotation of the second unit in a rotational direction from the first engagement position towards the final engagement position.

Some embodiments of the invention provide an apparatus comprising a first unit for connection by a bayonet fitting to a second unit in an electronic vapour provision system having a longitudinal axis. The bayonet fitting provides a mechanical and electrical connection between the first and second units. The first unit is arranged to: receive insertion of the second unit along said longitudinal axis to a first engagement position of the bayonet fitting; and allow the second unit to rotate about the longitudinal axis from the first engagement position to a final engagement position that prevents both (i) relative movement of the first and second units along said longitudinal axis, and (ii) rotation of the second unit past the final engagement position further away from the first engagement position. The first unit further includes in the bayonet fitting a biasing means that applies a rotational bias against rotation of the second unit from the final engagement position back to the first engagement position.

Some embodiments of the invention provide an electronic vapour provision system comprising first and second parts which when connected define a longitudinal axis, wherein the first and second parts are mechanically and electrically connectable via a bayonet fitting, the bayonet fitting providing co-axial and radial mechanical bias relative to the longitudinal axis.

The approach described herein is not restricted to specific embodiments such as set out above, but includes and contemplates any appropriate combinations of features presented in the whole document. For example, an electronic vapour provision system may be provided in accordance with the approach described herein which includes any one or more of the various features described above.

### Brief Description of the Drawings

Figure 1 is a schematic (exploded) diagram of an e-cigarette in accordance with some embodiments of the disclosure.
Figure 2 is a schematic diagram of the body of the e-cigarette of Figure 1 in accordance with some embodiments of the invention.
Figure 3 is a schematic diagram of the cartomiser of the e-cigarette of Figure 1 in accordance with some embodiments of the invention.
Figure 4 is a cross-section, in a plane parallel to the longitudinal axis of the e-cigarette, through the connector provided in the body of the e-cigarette of Figure 1 in accordance with some embodiments of the invention.
Figure 5 is a cross-section, in a plane perpendicular to the longitudinal axis of the e-cigarette, through the connector which joins the body and the cartomiser in the e-cigarette of Figure 1 at one stage of engagement in accordance with some embodiments of the invention.
Figure 6 is a cross-section, in a plane perpendicular to the longitudinal axis of the e-cigarette, through the connector which joins the body and the cartomiser in the e-cigarette of Figure 1 at a further stage of engagement in accordance with some embodiments of the invention.
Figure 7 is a schematic plot of two force curves showing the force exerted by a U-spring in the e-cigarette of Figure 1 at various points of engagement in accordance with some embodiments of the invention.

### Detailed Description

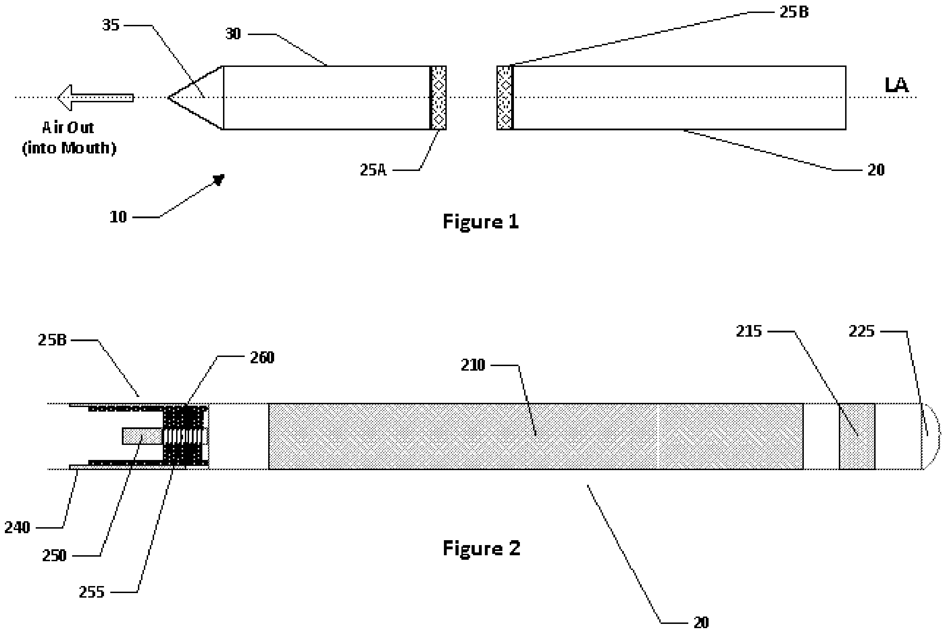

Figure 1 is a schematic diagram of an e-cigarette 10 in accordance with some embodiments of the invention (not to scale). The e-cigarette has a generally cylindrical shape, extending along a longitudinal axis indicated by dashed line LA, and comprises two main components, namely a body 20 and a cartomiser 30. The cartomiser includes an internal chamber containing a reservoir of nicotine, a vaporiser (such as a heater), and a mouthpiece 35. The reservoir may be a foam matrix or any other structure for retaining the nicotine until such time that it is required to be delivered to the vaporiser. The cartomiser 30 also includes a heater for vaporising the nicotine and may further include a wick or similar facility to transport a small amount of nicotine from the reservoir to a heating location on or adjacent the heater.

The body 20 includes a re-chargeable cell or battery to provide power to the e-cigarette 10 and a circuit board for generally controlling the e-cigarette. When the heater receives power from the battery, as controlled by the circuit board, the heater vaporises the nicotine and this vapour is then inhaled by a user through the mouthpiece.

The body 20 and cartomiser 30 are detachable from one another by separating along the longitudinal axis LA, as shown in Figure 1, but are joined together when the device 10 is in use by a connection, indicated schematically in Figure 1 as 25A and 25B, to provide mechanical and electrical connectivity between the body 20 and the cartomiser 30. The electrical connector on the body 20 that is used to connect to the cartomiser also serves as a socket for connecting a charging device (not shown) when the body is detached from the cartomiser 30. The other end of the charging device can be plugged into a USB socket to re-charge the cell in the body of the e-cigarette. In other implementations, a cable may be provided for direct connection between the electrical connector on the body and a USB socket.

The e-cigarette 10 is provided with one or more holes (not shown in Figure 1) for air inlet. These holes connect to an air passage through the e-cigarette 10 to the mouthpiece 35. When a user inhales through the mouthpiece 35, air is drawn into this air passage through the one or more air inlet holes, which are suitably located on the outside of the e-cigarette. This airflow (or the resulting change in pressure) is detected by a pressure sensor that in turn activates the heater to vaporise the nicotine from the cartridge. The airflow passes through, and combines with, the nicotine vapour, and this combination of airflow and nicotine vapour then passes out of the mouthpiece 35 to be inhaled by a user. The cartomiser 30 may be detached from the body 20 and disposed of when the supply of nicotine is exhausted (and replaced with another cartomiser if so desired).

It will be appreciated that the e-cigarette 10 shown in Figure 1 is presented by way of example, and various other implementations can be adopted. For example, in some embodiments, the cartomiser 30 is provided as two separable components, namely a cartridge comprising the nicotine reservoir and mouthpiece (which can be replaced when the nicotine from the reservoir is exhausted), and a vaporiser comprising a heater (which is generally retained). As another example, the charging facility may connect to an additional or alternative power source, such as a car cigarette lighter.

Figure 2 is a schematic (simplified) diagram of the body 20 of the e-cigarette of Figure 1 in accordance with some embodiments of the invention. Figure 2 can generally be regarded as a cross-section in a plane through the longitudinal axis LA of the e-cigarette. Note that various components and details of the body, e.g. such as wiring and more complex shaping, have been omitted from Figure 2 for reasons of clarity.

As shown in Figure 2, the body 20 includes a battery or cell 210 for powering the e-cigarette 10, as well as a chip, such as an application specific integrated circuit (ASIC) for controlling the e-cigarette 10. For example, the ASIC may include a sensor (or utilise a separate sensor, not shown in Figure 2) to detect an inhalation on mouthpiece 35, and to provide power from the battery 210 to a heater in the cartomiser (see below) to vaporise nicotine into the airflow which is inhaled by a user. The body further includes a cap 225 to seal and protect the far (distal) end of the e-cigarette.

At the opposite end of the body 20 from the cap 225 is the connector 25B for joining the body 20 to the cartomiser 30. In particular, the connector 25B provides mechanical and electrical connectivity between the body 20 and the cartomiser 30. The connector 25B includes a body connector 240, which is metallic (silver-plated in some embodiments) to serve as one terminal for electrical connection (positive or negative) to the cartomiser 30. The connector 25B further includes an electrical contact 250 to provide a second terminal for electrical connection to the cartomiser 30 of opposite polarity to the first terminal, namely body connector 240). The electrical contact 250 is mounted on a coil spring 255. When the body 20 is attached to the cartomiser 30, the connector 25A on the cartomiser pushes against the electrical contact 250 in such a manner as to compress the coil spring in an axial direction, i.e. in a direction parallel to (co-aligned with) the longitudinal axis LA. In view of the resilient nature of the spring 255, this compression biases the spring 255 to expand, which has the effect of pushing the electrical contact 250 firmly against connector 25A, thereby helping to ensure good electrical connectivity between the body 20 and the cartomiser 30. The body connector 240 and the electrical contact 250 are separated by a trestle 260, which is made of a non-conductor (such as plastic) to provide good insulation between the two electrical terminals. The trestle 260 is shaped as described below to assist with the mutual mechanical engagement of connectors 25A and 25B.

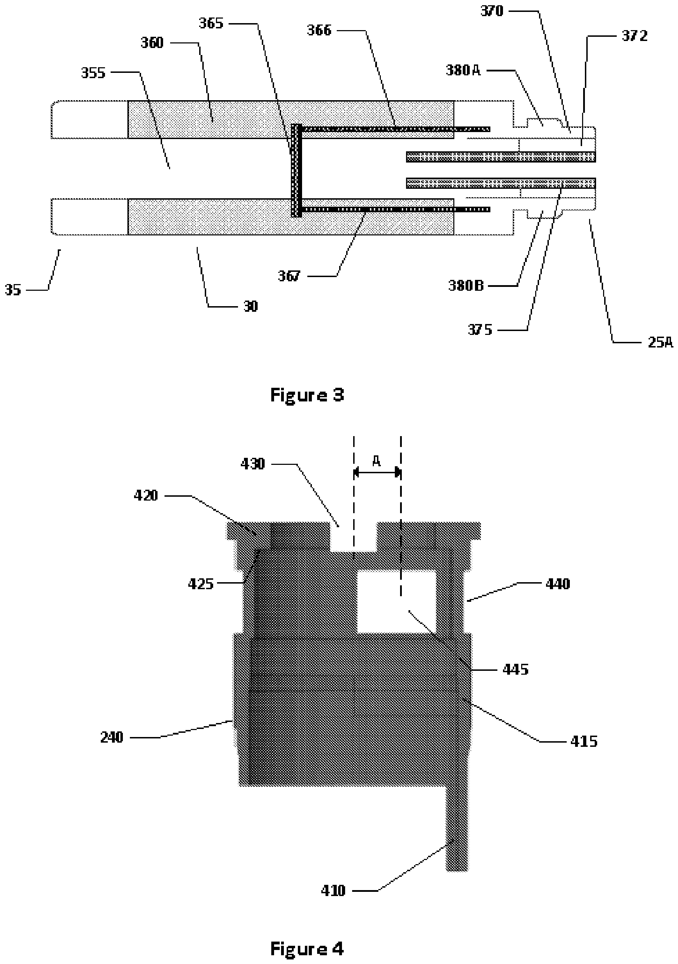

Figure 3 is a schematic diagram of the cartomiser 30 of the e-cigarette of Figure 1 in accordance with some embodiments of the invention. Figure 3 can generally be regarded as a cross-section in a plane through the longitudinal axis LA of the e-cigarette. Note that various components and details of the body, e.g. such as wiring and more complex shaping, have been omitted from Figure 3 for reasons of clarity.

The cartomiser 30 includes an air passage 355 extending along the central (longitudinal) axis of the cartomiser 30 from the mouthpiece 35 to the connector 25A for joining the cartomiser to the body 20. A reservoir of nicotine 360 is provided around the air passage 335. This reservoir 360 may be implemented, for example, by providing cotton or foam soaked in nicotine. The cartomiser also includes a heater 365 for heating nicotine from reservoir 360 to generate nicotine vapour to flow through air passage 355 and out through mouthpiece 35 in response to a user inhaling on the e-cigarette 10. The heater is powered through lines 366 and 367, which are in turn connected to opposing polarities (positive and negative, or vice versa) of the battery 210 via connector 25A (the details of the wiring between the power lines 366 and 367 and connector 25A are omitted from Figure 3).

The connector 25A includes an inner electrode 375, which may be silver-plated or made of some other suitable metal. When the cartomiser 30 is connected to the body 20, the inner electrode 375 contacts the electrical contact 250 of the body 20 to provide a first electrical path between the cartomiser and the body. In particular, as the connectors 25A and 25B are engaged, the inner electrode 375 pushes against the electrical contact 250 so as to compress the coil spring 255, thereby helping to ensure good electrical contact between the inner electrode 375 and the electrical contact 250.

The inner electrode 375 is surrounded by an insulating ring 372, which may be made of plastic, rubber, silicone, or any other suitable material. The insulating ring is surrounded by the cartomiser connector 370, which may be silver-plated or made of some other suitable metal or conducting material. When the cartomiser 30 is connected to the body 20, the cartomiser connector 370 contacts the body connector 240 of the body 20 to provide a second electrical path between the cartomiser and the body. In other words, the inner electrode 375 and the body connector 240 serve as positive and negative terminals (or vice versa) for supplying power from the battery 210 in the body to the heater 365 in the cartomiser via supply lines 366 and 367 as appropriate.

The cartomiser connector 370 is provided with two lugs or tabs 380A, 380B, which extended in opposite directions away from the longitudinal axis of the e-cigarette. These tabs are used to provide a bayonet fitting in conjunction with the body connector 240 for connecting the cartomiser 30 to the body 20. This bayonet fitting provides a secure and robust connection between the cartomiser 30 and the body 20, so that the cartomiser and body are held in a fixed position relative to one another, without wobble or flexing, and the likelihood of any accidental disconnection is very small. At the same time, the bayonet fitting provides simple and rapid connection and disconnection by an insertion followed by a rotation for connection, and a rotation (in the reverse direction) followed by withdrawal for disconnection.

Figure 4 is a schematic diagram of the body connector 240 of the e-cigarette of Figure 1 in accordance with some embodiments of the invention. The body connector 240 includes a wall 415 which has the general shape of a hollow cylinder (i.e. a tube or sleeve) which is co-aligned with respect to the longitudinal axis of the e-cigarette. Protruding from the wall at one end of the tube is an arm 410 which is used for inserting and fixing the body connector 240 into the body. At the opposite end of the tube, the wall 415 is provided with a lip or rim 420 that is inwardly directed (towards the central longitudinal axis). This inwardly-directed lip defines an aperture into hollow central portion of the body connector 240, and also creates an overhang 425 with respect to the main portion of the wall 415. In addition, the outside of the wall 415 (with respect to the central longitudinal axis) is provided with a shallow groove 440 that generally extends around the tube.

The path of the shallow groove 440 around the body connector 240 is interrupted by two windows or openings that extend through the wall 415 of the tube. One of these windows 445 is visible in Figure 4; the other window is substantially opposite to window 445 (with respect to the longitudinal axis of the e-cigarette 10). The path of the inwardly directed lip 420 is likewise interrupted by two openings that extend through the wall 415 of the tube. One of these openings 430 is visible in Figure 4; the other opening is substantially opposite to opening 430 (with respect to the longitudinal axis of the e-cigarette 10). If we take a centre-line through window 445 and a centre-line through opening 430 (each centre-line being parallel to the longitudinal axis of the e-cigarette 10), there is an offset between these two centre-lines. This offset is denoted as "A" in Figure 4, and corresponds to a given azimuthal rotation around the longitudinal axis of the e-cigarette 10.

When the cartomiser 30 is to be connected with body 20, they are held in coaxial alignment with one another, with connector 25A opposite to connector 25B. The connector 25A, which is the male portion of the bayonet fitting, is then inserted into connector 25B, the female portion of the bayonet fitting. More particularly, the end of the cartomiser connector 370 is inserted into the central hole of body connector 240, i.e. within wall 415, through the circular aperture defined by lip 420.

After an initial insertion, the lugs 380A, 380B extending radially outwards from the cartomiser connector 370 contact the end of the body connector 240, more particularly, lip 420. This contact prevents further insertion of the cartomiser connector 370 into the body connector 240 until (or unless) the cartomiser 30 and the body 20 are aligned in an azimuthal direction (by relative rotation about the longitudinal axis of the e-cigarette) so that the two lugs 380A, 380B coincide with respective openings 430 in the lip 420. The openings 430 allow the lugs 380A, 380B to pass through the lip 420, thereby enabling further insertion of the cartomiser connector 370 into the body connector 240.

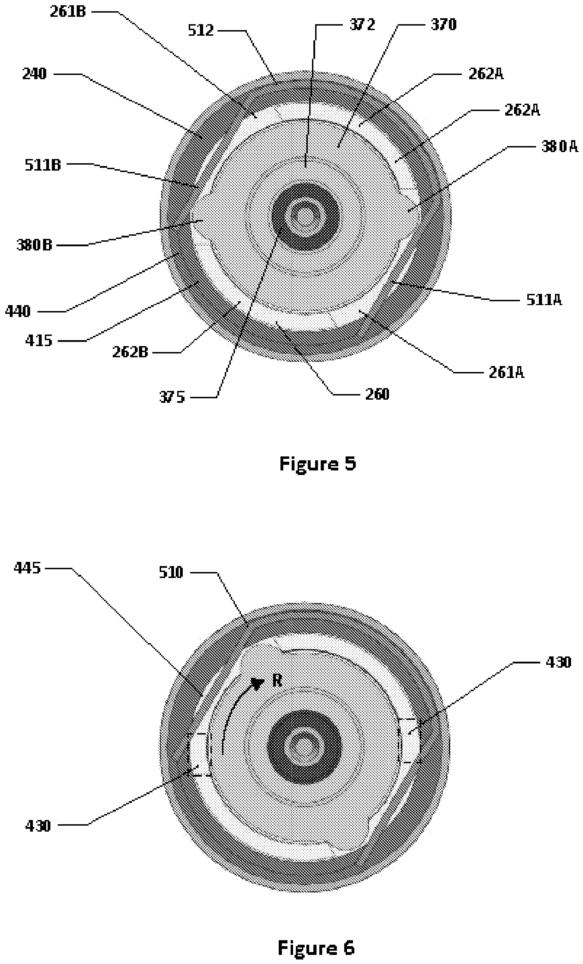

Figure 5 and 6 are cross-sections through the e-cigarette of Figure 1 in a plane perpendicular to the longitudinal axis of the e-cigarette in a direction looking into the body 20, in accordance with some embodiments of the invention. The cross-sections are taken through the connectors 25A, 25B after they are engaged by one another by inserting the cartomiser 30 into the body 20 (allowing for any rotation necessary to align the lugs 380A, 380B with respective openings 430 in the lip 420).

As can be seen from the cross-section of Figure 5, once the lugs 380A, 380B have passed through the openings 430 in the lip 420 of body connector 240, the inner diameter of the body connector wall 415 is sufficiently large to allow rotation of the lugs 380A and 380B with respect to the longitudinal axis of the e-cigarette, as well as motion further along the longitudinal axis - i.e. in a direction to further insert the connector 25A of the cartomiser 30 into the connector 25B of the body 20. This further insertion however is limited by the trestle 260. The portion of the trestle 260 shown in Figure 5 has the general shape of a hollow cylinder (i.e. a tube or sleeve) with respect to the longitudinal axis of the e-cigarette, and sits or nests immediately inside the body connector 240. The top of the trestle, i.e. the end closest to the cartomiser 30 after engagement, sits immediately below the rim 420 of the body connector 240, in other words, abutting the underside face 425 of the rim 420.

It will be appreciated that this positioning of the trestle would prevent insertion of the the lugs 380A, 380B past the lip 420 of body connector 440, except that in two opposing regions 261A, 261B the topmost portion of the trestle 260 is removed to a predetermined depth. Accordingly, only the two remaining (intervening) topmost portions 262A and 262B of the trestle abut against the underside face 425 of the rim 420. The cutaway regions 261A, 261B have a depth corresponding to the size of the lugs 380A, 380B. In addition, the trestle 260 and the body connector 240 are mutually aligned in an azimuthal direction such that one end of each cutaway is below (aligned with) a respective opening 430 of the lip 420. The locations of these openings 430 in the lip 420 relative to the cutaway regions 261A, 261B are indicated schematically by the dashed line in Figure 6.

In operation therefore, further insertion of lugs 380A, 380B through the openings 430 of the lip 420 of body connector 240 is permitted by respective cutaway regions 261A and 261B until the lugs 380A and 380B contact the top of the trestle 260 within these cutaway regions 261A, 261B. At this point the position has been reached as shown in Figure 5, which will be referred to as a first point of engagement between the body 20 and the cartomiser 30. At this first point of engagement, the trestle 240 prevents any further (longitudinal) insertion of the body 20 into cartomiser 30. However, the cutaway regions 261A, 261B do define a channel between the top of the trestle and the underside face 425 of the rim 420 of the body connector 240 to allow relative rotation of the connectors 25A and 25B. In particular, the connector 25A and/or the connector 25B can be rotated to move the lugs 380A, 380B along this channel, in the direction indicated by arrow R in Figure 6, until the lugs reach the end of respective the cutaway regions 261A, 261B and abut against retained wall regions 262A, 262B respectively. At this point the position has been reached as shown in Figure 6, which will be referred to as a second or final point of engagement between the body 20 and the cartomiser 30. Note that in this second point of engagement, the lugs 380A, 380B are unable to move along the longitudinal axis, since they are constrained in one direction by the overhang 425 of the rim 420, and in the opposite direction by the floor of the cutaway regions 261A, 261B of the trestle 260. In addition, the lugs are prevented from further rotation (past the second point of engagement is a direction away from the first point of engagement) by the retained wall portions 262A, 262B.

In fact, the lugs are slightly biased against the overhang 425 of the rim 420 by the coil spring 255, since this tends to push the connector 25A (including lugs 380A, 380B) away from the body 20. This bias is resisted by the rim 420 of the body connector 240. Accordingly, the connectors 25A, 25B provide a bayonet fitting to retain the cartomiser 30 in engagement with the body 20.

The connector 25B includes a further component, namely U-spring 510. This U-spring comprises two arms 511A and 511B, which are substantially straight, and which are joined by an arc section 512. The U-spring 510 is resilient in that the arms can be separated by using an appropriate force, but are then mechanically biased to return to their original position by the elasticity of the arc section trying to regain its original curvature.

The U-spring is positioned on the outside of the body connector 240. In particular, the U-spring is retained in a longitudinal direction by groove 440 in the outer wall of the body connector. The U-spring is positioned in an azimuthal direction by the location of windows 445, since the straight arms 511A, 511B are accommodated by (sit across) these windows, as shown in Figures 5 and 6.

It will be appreciated that as the connectors 25A and 25B are rotated from the first engagement position of Figure 5 to the second engagement position of Figure 6, the lugs 380A, 380B have, in effect, to push past the respective straight arms 511A, 511B. Accordingly, the straight arms 511A, 511B must be deflected or distorted outwards to allow movement of the lugs from the first to second engagement positions, or vice versa, which in turn requires a force (moment or torque) to overcome the resilience of the U-spring 510.

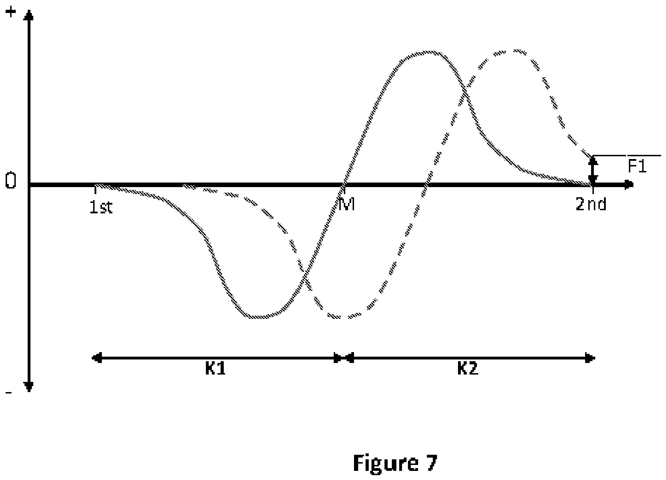

Figure 7 is a schematic diagram illustrating the rotational force or bias (solid line) that the lugs 380A, 380B experience from the U-spring 510 as the connectors 25A and 25B are rotated from the first engagement position to the second engagement position or vice versa. In Figure 7, a positive force is directed in the clockwise direction of Figures 5 and 6, i.e. from the first engagement position to the second engagement position, as indicated by arrow R in Figure 6, while a negative force is directed in the opposite direction, i.e. from the second engagement position back to the first engagement position.

As shown in Figure 7, as the connectors 25A and 25B are rotated from the first engagement position to the second engagement position, the lugs initially experience a negative force, i.e. resisting the rotation from the first engagement position to the second engagement position. This region of negative force is denoted K1 in Figure 7. However, once a transition point M has been reached, which is when the motion of the lugs 380A, 380B is substantially tangential to the respective arms 511A, 511B of the U-spring 510, the sign of the rotational force changes to become positive. For the region K2 beyond the transition point M, the U-spring 510 therefore contributes to urging the connectors 25A and 25B into the second engagement position.

When removing (disengaging) the body 20 from the cartomiser 30, the opposite rotation of the connectors 25A and 25B must occur, namely from the second engagement position to the first engagement position. This then aligns the lugs 380A, 380B with the openings 430 in the body connector 240, thereby permitting the cartomiser connector 370 to be withdrawn from the body connector 240. Note that in rotating from the second engagement position to the first engagement position, the connector 25A must first pass through region K2 in which the U-spring 510 provides a positive force - i.e. in a direction to resist motion from the second engagement position to the first engagement position. This resistance therefore provides a form of locking for the bayonet connection, since the U-spring helps to prevent accidental disengagement of the cartomiser 30 from the body 20. In particular, such disengagement in the e-cigarette 10 can only occur if a sufficient rotational force is applied to overcome this initial resistance of the U-spring 510. Once this resistance has been overcome, and the rotation has reached the transition point M, the sign of the force from the U-spring 510 changes, and it urges the connectors 25A, 25B to rotate in the direction back to the first engagement position. In this position, the lugs 380A, 380B are aligned with the openings 430 in the body connector 240, thereby permitting withdrawal of the cartomiser 30 from the body 20.

Figure 7 also illustrates the rotational force (dashed line) in an alternative arrangement in which the U-spring is slightly rotated compared with the position shown in Figures 5 and 6 (in a clockwise direction). This rotation can be accomplished by moving the windows 445 relative to the openings 430 and the position of the trestle 260 (which both remain as shown in Figures 5 and 6). In other words, the alternative arrangement has a different value of the offset angle A shown in Figure 4 between the windows 445 and the openings 430.

This slight change in location of the U-spring 510 induces in effect a corresponding sideways translation of the force curve relative to the first and second engagement positions (which can be considered as defined by the cutaway portions 261A, 261B of the trestle). In particular, the dashed line force curve generally follows the solid line curve discussed above, but shifted to the right (in the rotational direction from the first engagement position to the second engagement position). This shift results in the U-spring continuing to exert a positive rotational force (indicated as F1 in Figure 7) on the lugs 380A, 380B when the connectors 25A, 25B are in the second engagement position. This residual force in the positive azimuthal (rotational) direction acts to hold the lugs 380A, 380B against the sides of the respective remaining wall portions 262A, 262B which prevent further rotation of the lugs 380A, 380B in the direction of arrow R (see Figure 6).

Compared with the force curve shown in solid line, the force curve shown in the dashed line of Figure 7 helps to prevent any slight relative wobble of the body 20 and cartomiser 30 in an azimuthal direction, in particular away from the second engagement position towards the first engagement position (i.e. in the opposite direction to arrow R), since the U-spring 510 in effect biases the cartomiser connector 370 into the second engagement position. Accordingly, setting the offset angle A of Figure 4 to position the U-spring 510 so that it provides the dashed force line of Figure 7 helps to achieve a more secure and robust mechanical and electrical connection for e-cigarette 10.

Note that as the connectors 25A and 25B are rotated from the first engagement position to the second engagement position, energy is saved into the U-spring in region K1 and then released in region K2. In other words, having a user rotate the connectors 25A, 25B in region K1 against the opposing force of the U-spring 510 provides the energy to place the U-spring 510 into a biased configuration, which is then used to hold cartomiser connector 370 in the second engagement position. It will also be noted that the mechanical biasing force provided by the U-spring 150 is primarily in a radial direction (towards the longitudinal axis). However, if the points of contact between the lugs 380A, 380B and the corresponding arms 511A, 511B are in positions where the arms 511A, 511B are not completely tangential (perpendicular) to the radial direction, which may arise in part because of an outward deflection of arms 511A, 511B by lugs 380A, 380B, then a component of this radial force is converted into a rotation force, which can be used as described above to retain the connectors 25A, 25B in their final engagement position.

In addition, note that Figure 7 does not incorporate any friction arising from motion of the lugs against the surface of the spring 510. However, a component of the biasing force from the U-spring 510 is normal to the point of contact between the lugs 380A, 380B and the respective arms 511A, 511B of the U-spring. This biasing force, in a substantially radial or normal direction, may cause increased friction between the lugs 380A, 380B and the respective arms 511A, 511B of the spring 510, and this increased friction may also help to prevent wobble of the cartomiser connector 370 when located in the second engagement position.

Although the embodiments discussed above illustrate the use of U-spring 510 to provide a bias for the bayonet fitting, it will be appreciated that many other mechanisms or approaches are available for performing such a function. For example, rather than utilising the curved segment 512 of the U-spring 510 to provide an elastic or biasing force, each window 445 might be individually fitted with a biasing member, analogous to the two U-spring arms 511, but omitting curved segment 512 so that the biasing members are not joined together. The biasing members are then elastically deformed by the rotation of lugs 380A, 380B (or any other suitable form of actuating component) - for example, the biasing members could comprise a straight or curved strip of material (such as rubber) that is compressed by the rotation of lugs 380A, 380B, or a straight or curved strip of resilient material (such as metal or plastic) that is deflected outwards by the rotation of lugs 380A, 380B.

The configuration illustrated in the Figures above provides the biasing members on the outside (in a radial direction) of the lugs 380A, 380B. In other configurations however, the biasing member(s) may be provided on the inside (in a radial direction) of some suitable actuating component, or alternatively they may be offset from the actuating component in a longitudinal direction, such that the compression, deflection or other elastic distortion occurs primarily in a direction parallel to the longitudinal axis. Furthermore, the total number of biasing members may vary from one configuration to another (e.g. one, two, three, four or more biasing members may be present). Accordingly, the biasing member(s) and the corresponding actuating member(s) may have any suitable shape, position and configuration for providing an appropriate biasing force or torque.

In addition, the allocation of the different components of the bayonet fitting, including such biasing member(s) and the corresponding actuating member(s), to the different components of the electronic vapour provision systems may be different from that shown in the above embodiments. Note also that although the various components may be sold together as a complete electronic vapour provision system, in some cases the different components may be sold individually, for example, as replacement unit if the nicotine in a cartomiser is exhausted.

In order to address various issues and advance the art, this disclosure shows by way of illustration various embodiments in which the claimed invention(s) may be practiced. The advantages and features of the disclosure are of a representative sample of embodiments only, and are not exhaustive and/or exclusive. They are presented only to assist in understanding and to teach the claimed invention(s). It is to be understood that advantages, embodiments, examples, functions, features, structures, and/or other aspects of the disclosure are not to be considered limitations on the disclosure as defined by the claims or limitations on equivalents to the claims, and that other embodiments may be utilised and modifications may be made without departing from the scope of the claims. Various embodiments may suitably comprise, consist of, or consist essentially of, various combinations of the disclosed elements, components, features, parts, steps, means, etc other than those specifically described herein.

## Claims

1. An electronic vapour provision system (10) comprising first and second units which when connected define a longitudinal axis, wherein the first and second units are mechanically and electrically connectable via a bayonet fitting (25), the bayonet fitting providing co-axial and radial mechanical bias relative to the longitudinal axis; wherein the first unit is arranged to:
receive insertion of the second unit along said longitudinal axis to a first engagement position of the bayonet fitting; and
allow the second unit to rotate about the longitudinal axis from the first engagement position to a final engagement position that prevents both (i) relative movement of the first and second units along said longitudinal axis, and (ii) rotation of the second unit past the final engagement position further away from the first engagement position;
**characterized in that** the first unit further includes in the bayonet fitting a biasing means (510) that applies a rotational bias against rotation of the second unit from the final engagement position back to the first engagement position, wherein a transition point is located between the first engagement position and the final engagement position, and when the second unit is located between the first engagement position and the transition point, the biasing means applies a rotational bias in favour of rotation of the second unit in a rotational direction from the final engagement position towards the first engagement position, and when the second unit is located between the final engagement position and the transition point, the biasing means applies a rotational bias in favour of rotation of the second unit in a rotational direction from the first engagement position towards the final engagement position.

2. The electronic vapour provision system of claim 1, wherein the biasing means suffers elastic deformation as the second unit is rotated between the first engagement position and the final engagement position, and wherein the elastic deformation is a maximum at said transition point.

3. The electronic vapour provision system of claim 2, wherein said elastic deformation is primarily in a radial direction with respect to the longitudinal axis.

4. The electronic vapour provision system of claim 3, wherein said elastic deformation is caused by at least one protruding member on the second unit (380), and wherein said transition point occurs when the biasing means has a tangential configuration with respect to the rotation of said at least one protruding member.

5. The electronic vapour provision system of claim 4, wherein said biasing means comprises a substantially U-shaped member having a pair of opposing arms (511A, 511B) joined together by an arc section (512), wherein said a rotational bias against rotation of the second unit from the final engagement position back to the first engagement position is caused by separation of the arms from a rest position.

6. The electronic vapour provision system of claim 5, wherein the arc section is substantially coaxial with said longitudinal axis.

7. The electronic vapour provision system of claim 6, wherein the arc section is rotationally located with respect to said first and final engagement positions such that said rotational bias is applied when the first and second units are in the final engagement position.

8. The electronic vapour provision system of claim 7, wherein the first unit includes a connector (25B) which includes:
one or more channels (440) for receiving a respective protruding member of the second unit; and
a pair of windows (445) for receiving a respective opposing arm of said substantially U-shaped member, wherein the second unit has two protruding members (380A, 380B) that contact the biasing means through a respective window;
and optionally wherein the one or more channels for receiving a respective protruding member of the second unit act to guide the second unit as it is inserted into the first unit to the first engagement position and then rotated from the first engagement position to the final engagement position.

9. The electronic vapour provision system of any preceding claim, wherein said first unit comprises a body portion (20) of the electronic vapour provision system, said body portion including a cell or battery (230) and a control circuit.

10. The electronic vapour provision system according to claim 9, wherein said second unit comprises a cartomiser, further wherein said cartomiser includes a reservoir (360) of a substance and a heater (365) powered by the cell or battery of said body portion for vaporising said substance.

11. The electronic vapour provision system of any of claims 1 to 10, wherein said rotational bias is applied when the first and second units are in the final engagement position.

## Patentansprüche

1. Elektronisches Dampfbereitstellungssystem (10), umfassend erste und zweite Einheiten, die bei Verbindung eine Längsachse definieren, wobei die ersten und zweiten Einheiten mechanisch und elektrisch über einen Bajonettanschluss (25) verbindbar sind, wobei der Bajonettanschluss koaxiale und radiale mechanische Vorspannung relativ zur Längsachse bereitstellt; wobei die erste Einheit angeordnet ist zum
Aufnehmen des Einsetzens der zweiten Einheit entlang der Längsachse in eine erste Eingriffsposition des Bajonettanschlusses; und
Ermöglichen der Drehung der zweiten Einheit um die Längsachse aus der ersten Eingriffsposition in eine abschließende Eingriffsposition, die sowohl (i) relative Bewegung der ersten und zweiten Einheiten um die Längsachse als auch (ii) Drehung der zweiten Einheit hinter die von der ersten Eingriffsposition weiter entfernte abschließende Eingriffsposition verhindert;
**dadurch gekennzeichnet, dass** die erste Einheit im Bajonettanschluss ferner ein Vorspannmittel (510) aufweist, das eine Dreh-Vorspannung gegen die Drehung der zweiten Einheit aus der abschließenden Eingriffsposition zurück zur ersten Eingriffsposition beaufschlagt, wobei ein Übergangspunkt zwischen der ersten Eingriffsposition und der abschließenden Eingriffsposition angeordnet ist und, wenn die zweite Einheit zwischen der ersten Eingriffsposition und dem Übergangspunkt angeordnet ist, das Vorspannmittel eine Dreh-Vorspannung zugunsten der Drehung der zweiten Einheit in einer Drehrichtung von der abschließenden Eingriffsposition in Richtung auf die erste Eingriffsposition beaufschlagt, und wenn die zweite Einheit zwischen der abschließenden Eingriffsposition und dem Übergangspunkt angeordnet ist, das Vorspannmittel eine Dreh-Vorspannung zugunsten der Drehung der zweiten Einheit in einer Drehrichtung von der ersten Eingriffsposition in Richtung auf die abschließende Eingriffsposition beaufschlagt.

2. Elektronisches Dampfbereitstellungssystem nach Anspruch 1, wobei das Vorspannmittel elastische Verformung erfährt, wenn die zweite Einheit zwischen der ersten Eingriffsposition und der abschließenden Eingriffsposition gedreht wird, und wobei die elastische Verformung an dem Übergangspunkt ein Maximum erreicht.

3. Elektronisches Dampfbereitstellungssystem nach Anspruch 2, wobei die elastische Verformung primär in einer radialen Richtung mit Bezug auf die Längsachse auftritt.

4. Elektronisches Dampfbereitstellungssystem nach Anspruch 3, wobei die elastische Verformung durch mindestens ein hervorstehendes Element an der zweiten Einheit (380) bewirkt wird und wobei der Übergangspunkt auftritt, wenn das Vorspannmittel mit Bezug auf die Drehung des mindestens einen hervorstehenden Elements eine Tangentialkonfiguration aufweist.

5. Elektronisches Dampfbereitstellungssystem nach Anspruch 4, wobei das Vorspannmittel ein im Wesentlichen U-förmiges Element umfasst, aufweisend ein Paar gegenüberliegender Arme (511A, 511B), die durch einen Bogenabschnitt (512) miteinander verbunden werden, wobei die Dreh-Vorspannung gegen Drehung der zweiten Einheit von der abschließenden Eingriffsposition zurück zur ersten Eingriffsposition durch Lösen der Arme aus einer Ruheposition bewirkt wird.

6. Elektronisches Dampfbereitstellungssystem nach Anspruch 5, wobei der Bogenabschnitt im Wesentlichen koaxial mit der Längsachse ist.

7. Elektronisches Dampfbereitstellungssystem nach Anspruch 6, wobei der Bogenabschnitt in Drehungsposition mit Bezug auf die erste und auf die abschließende Eingriffsposition angeordnet ist, sodass die Dreh-Vorspannung beaufschlagt wird, wenn die ersten und zweiten Einheiten in der abschließenden Eingriffsposition sind.

8. Elektronisches Dampfbereitstellungssystem nach Anspruch 7, wobei die erste Einheit einen Verbindungsanschluss (25B) aufweist, der aufweist:
einen oder mehrere Kanäle (440) zum Aufnehmen eines entsprechenden hervorstehenden Elements der zweiten Einheit; und
ein Paar Fenster (445) zum Aufnehmen eines entsprechenden gegenüberliegenden Arms des im Wesentlichen U-förmigen Elements, wobei die zweite Einheit zwei hervorstehende Elemente (380A, 380B) aufweist, die das Vorspannmittel durch ein entsprechendes Fenster kontaktieren;
und wobei optional der eine Kanal oder die mehreren Kanäle zum Aufnehmen eines entsprechenden hervorstehenden Elements der zweiten Einheit wirken, um die zweite Einheit zu führen, während sie in die erste Einheit bis zur ersten Eingriffsposition eingesetzt und anschließend aus der ersten Eingriffsposition zur abschließenden Eingriffsposition gedreht wird.

9. Elektronisches Dampfbereitstellungssystem nach einem der vorstehenden Ansprüche, wobei die erste Einheit einen Körperabschnitt (20) des elektronischen Dampfbereitstellungssystems umfasst, wobei der Körperabschnitt eine Zelle oder Batterie (230) und einen Steuerschaltkreis umfasst.

10. Elektronisches Dampfbereitstellungssystem nach Anspruch 9, wobei die zweite Einheit einen Cartomizer umfasst, wobei ferner der Cartomizer einen Behälter (360) einer Substanz und ein Heizelement (365) aufweist, mit Energie versorgt durch die Zelle oder Batterie des Körperabschnitts zum Verdampfen der Substanz.

11. Elektronisches Dampfbereitstellungssystem nach einem der Ansprüche 1 bis 10, wobei die Dreh-Vorspannung beaufschlagt wird, wenn die ersten und zweiten Einheiten in der abschließenden Eingriffsposition sind.

## Revendications

1. Système électronique de fourniture de vapeur (10) comprenant des première et seconde unités qui, lorsqu'elles sont reliées, définissent un axe longitudinal, dans lequel les première et seconde unités peuvent être reliées mécaniquement et électriquement par l'intermédiaire d'un raccord à baïonnette (25), le raccord à baïonnette permettant une sollicitation mécanique co-axiale et radiale par rapport à l'axe longitudinal ; dans lequel la première unité est conçue pour :
recevoir une insertion de la seconde unité le long dudit axe longitudinal vers une première position d'entrée en prise du raccord à baïonnette ; et
permettre que la seconde unité tourne autour de l'axe longitudinal depuis la première position d'entrée en prise vers une position d'entrée en prise finale qui évite à la fois (i) un mouvement relatif des première et seconde unités le long dudit axe longitudinal et (ii) la rotation de la seconde unité au-delà de la position d'entrée en prise finale plus loin que la première position d'entrée en prise ;
**caractérisé en ce que** la première unité comprend en outre dans le raccord à baïonnette un moyen de sollicitation (510) qui applique une sollicitation rotative à l'encontre de la rotation de la seconde unité depuis la position d'entrée en prise finale de retour vers la première position d'entrée en prise, un point de transition étant situé entre la première position d'entrée en prise et la position d'entrée en prise finale, et lorsque la seconde unité est située entre la première position d'entrée en prise et le point de transition, le moyen de sollicitation appliquant une sollicitation rotative en faveur d'une rotation de la seconde unité dans une direction rotative depuis la position d'entrée en prise finale vers la première position d'entrée en prise, et lorsque la seconde unité est située entre la position d'entrée en prise finale et le point de transition, le moyen de sollicitation appliquant une sollicitation rotative en faveur d'une rotation de la seconde unité dans une direction rotative depuis la première position d'entrée en prise vers la position d'entrée en prise finale.

2. Système électronique de fourniture de vapeur selon la revendication 1, dans lequel le moyen de sollicitation subit une déformation élastique à mesure que la seconde unité est mise à tourner entre la première position d'entrée en prise et la position d'entrée en prise finale, et dans lequel la déformation élastique est un maximum au niveau dudit point de transition.

3. Système électronique de fourniture de vapeur selon la revendication 2, dans lequel ladite déformation élastique est principalement dans une direction radiale par rapport à l'axe longitudinal.

4. Système électronique de fourniture de vapeur selon la revendication 3, dans lequel ladite déformation élastique est provoquée par au moins un élément saillant sur la seconde unité (380), et dans lequel ledit point de transition se produit lorsque le moyen de sollicitation a une configuration tangentielle par rapport à la rotation dudit au moins un élément saillant.

5. Système électronique de fourniture de vapeur selon la revendication 4, dans lequel ledit moyen de sollicitation comprend un élément sensiblement en forme de U possédant une paire de bras opposés (511A, 511B) joints ensemble par une section arquée (512), dans lequel ladite sollicitation rotative à l'encontre de la rotation de la seconde unité depuis la position d'entrée en prise finale de retour vers la première position d'entrée en prise est provoquée par la séparation des bras depuis une position de repos.

6. Système électronique de fourniture de vapeur selon la revendication 5, dans lequel la section arquée est sensiblement coaxiale avec ledit axe longitudinal.

7. Système électronique de fourniture de vapeur selon la revendication 6, dans lequel la section arquée est située rotative par rapport auxdites première position d'entrée en prise et position d'entrée en prise finale de sorte que ladite sollicitation rotative soit appliquée lorsque les première et seconde unités sont dans la position d'entrée en prise finale.

8. Système électronique de fourniture de vapeur selon la revendication 7, dans lequel la première unité comprend un raccord (25B) qui comprend :
un ou plusieurs canaux (440) pour recevoir un élément saillant respectif de la seconde unité ; et
une paire de fenêtres (445) pour recevoir un bras opposé respectif dudit élément sensiblement en forme de U, la seconde unité possédant deux éléments saillants (380A, 380B) qui entrent en contact avec le moyen de sollicitation à travers une fenêtre respective ;
et éventuellement dans lequel le ou les canaux pour recevoir un élément saillant respectif de la seconde unité agissent pour guider la seconde unité à mesure qu'elle est insérée dans la première unité vers la première position d'entrée en prise puis mise à tourner depuis la première position d'entrée en prise vers la position d'entrée en prise finale.

9. Système électronique de fourniture de vapeur selon l'une quelconque des revendications précédentes, dans lequel ladite première unité comprend une partie corps (20) du système électronique de fourniture de vapeur, ladite partie corps comprenant une pile ou une batterie (230) et un circuit de commande.

10. Système électronique de fourniture de vapeur selon la revendication 9, dans lequel ladite seconde unité comprend un cartomiseur, en outre dans lequel ledit cartomiseur comprend un réservoir (360) d'une substance et un élément chauffant (365) alimenté par la pile ou la batterie de ladite partie corps pour vaporiser ladite substance.

11. Système électronique de fourniture de vapeur selon l'une quelconque des revendications 1 à 10, dans lequel ladite sollicitation rotative est appliquée lors les première et seconde unités sont dans la position d'entrée en prise finale.
